(19) **Europäisches Patentamt / European Patent Office / Office européen des brevets**

(11) **EP 4 498 122 A1**

(12) # DEMANDE DE BREVET EUROPEEN

(43) Date de publication:
**29.01.2025 Bulletin 2025/05**

(21) Numéro de dépôt: **24186943.7**

(22) Date de dépôt: **05.07.2024**

(51) Classification Internationale des Brevets (IPC):
**G01S 7/52** (2006.01)   **B06B 1/02** (2006.01)

(52) Classification Coopérative des Brevets (CPC):
**G01S 7/5202; B06B 1/0215;** B06B 2201/76

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **27.07.2023 FR 2308112**

(71) Demandeur: **SUPERSONIC IMAGINE**
**13290 Aix-en-Provence (FR)**

(72) Inventeurs:
• **Marsac, Laurent**
  **13090 Aix-en-Provence (FR)**
• **Dolgoff, Cyril**
  **83560 Ginasservis (FR)**
• **Zhang, Bo**
  **13109 Simiane-Collongue (FR)**
• **Frappart, Thomas**
  **13090 Aix-en-Provence (FR)**

(74) Mandataire: **RVDB Nantes**
**2, rue Crucy**
**CS 60515**
**44005 Nantes Cedex 1 (FR)**

(54) **PROCÉDÉ ET DISPOSITIF DE TRAITEMENT D'UN SIGNAL ÉLECTRIQUE POUR DISPOSITIF ÉMETTEUR ULTRASONORE**

(57)    La présente divulgation concerne un dispositif (10) et un procédé de traitement d'un signal électrique (SG1) pour un dispositif émetteur ultrasonore (20). Le procédé comprend : génération d'un signal électrique (SG1) définissant des ondes ultrasonores (W), la périodicité du signal électrique (SG1) étant modifiée par une modulation (ML1) de la phase du signal (PH1) ; et fourniture du signal électrique (SG1) au dispositif émetteur ultrasonore (20) pour causer l'émission des ondes ultrasonores (W).

## Fig.3

## Description

## Technique antérieure

Un dispositif émetteur ultrasonore (ou dispositif d'émission ultrasonore) comprend habituellement une pluralité d'éléments transducteurs configurés pour émettre des ondes ultrasonores, à des fins diverses, par exemple pour réaliser de l'imagerie par ultrasons.

A cet effet, le dispositif émetteur ultrasonore peut être piloté au moyen de signaux électriques, ces derniers pouvant être émis au moyen par exemple d'un ou des pulseurs ou d'amplificateurs linéaires. Ces signaux électriques définissent des ondes transmises aux éléments transducteurs du dispositif émetteur, causant ainsi l'émission d'ondes ultrasonores dans un milieu donné. Des signaux électriques peuvent éventuellement être produits en retour par ces mêmes éléments transducteurs (ou d'autres éléments transducteurs), ces signaux représentant une réponse (ou écho) du milieu aux sollicitations ondulatoires.

En général, en cours de fonctionnement, un tel dispositif émetteur ultrasonore émet des ondes électromagnétiques (EM). A ce titre, le dispositif émetteur ultrasonore constitue également un dispositif émetteur d'ondes électromagnétiques (dit aussi dispositif émetteur EM). Un fort rayonnement d'ondes électromagnétiques n'est cependant pas toujours souhaitable.

Il existe ainsi différentes normes qui imposent des niveaux limites autorisés d'émissions électromagnétiques des appareils électriques, scientifiques et médicaux, telles que par exemple les normes IEC 60601-1-2, NF EN 55011 / CISPR 11 et IEC 61000-4-3.

En particulier, la norme CISPR 11 (cf. par exemple l'édition 6.2 2019-01) définit les limites autorisées (c'est-à-dire les valeurs « quasi-peak »/« quasi-crêtes » de l'émission électromagnétique) pour différentes plages de fréquences utilisées. En conséquence, l'énergie d'un champ électrique émis par un dispositif ne doit pas dépasser ces limites pour pouvoir être déclaré comme dispositif conforme à ladite norme. Par exemple, le tableau 6 de la norme CISPR 11 concerne les limites de perturbation par rayonnement électromagnétique pour les appareils de classe A groupe 1, mesurées sur un site d'essai. De plus, le tableau 2 de la norme CISPR 11 concerne les limites de tension perturbatrice pour les appareils de classe A groupe 1, mesurées sur un site d'essai. Un dispositif médical, comme par exemple un système comprenant une sonde à ultrasons, est typiquement de classe A, dans la mesure où il s'agit d'un appareil non-domestique.

En outre, le standard IEC 61000-4-3 / EN 61000-4-3 définit une norme de compatibilité électromagnétique (CEM). Ses parties 4-3 notamment concernent les techniques d'essai et de mesure utilisées, ainsi que l'essai d'immunité aux champs électromagnétiques rayonnés aux fréquences radioélectriques.

Les dispositifs ultrasonores émettant par construction des ondes électromagnétiques, ils doivent rester compatibles avec lesdites normes, tout en autorisant un usage optimal, c'est à dire avec émission d'ondes utiles pour l'étude de région d'intérêt concernée.

## Exposé de la divulgation

Comme indiqué ci-avant, les dispositifs émetteurs ultrasonores conventionnels posent notamment problème en ce qu'ils peuvent être source d'émissions électromagnétiques qu'il peut être souhaitable de limiter, par exemple dans leur rayonnement, leurs directions et/ou leurs intensités.

L'un des objets de la présente divulgation est de résoudre au moins l'un des problèmes ou déficiences décrits précédemment.

En particulier, un objet de la présente divulgation est de limiter les émissions électromagnétiques d'un dispositif émetteur ultrasonore tout en assurant à ce dernier des performances satisfaisantes, en termes par exemple de réponse spectrale et/ou d'énergie (ou puissance) acoustique délivrée.

En particulier, un objet de la présente divulgation est de concevoir un dispositif émetteur ultrasonore performant, générant un minimum d'émission électromagnétique en fonctionnement tout en présentant une complexité de conception, fabrication et d'utilisation limitée.

A cet effet, selon un premier aspect, la présente divulgation vise un procédé de traitement d'un signal électrique pour un dispositif émetteur ultrasonore, ledit procédé comprenant :

- génération d'un signal électrique définissant des ondes ultrasonores, la périodicité du signal électrique étant modifiée par une modulation de la phase du signal ; et
- fourniture du signal électrique au dispositif émetteur ultrasonore pour causer l'émission des ondes ultrasonores.

En mettant en oeuvre un tel procédé, il devient avantageusement possible de limiter les rayonnements électromagnétiques émis par un dispositif émetteur ultrasonore tout en assurant à ce dernier des performances satisfaisantes, en termes par exemple de réponse spectrale et/ou d'énergie (ou puissance) acoustique délivrée. On peut ainsi avantageusement limiter le niveau des émissions électromagnétiques avec un minimum d'impact (idéalement aucun impact) sur les performances de fonctionnement souhaitées du dispositif émetteur ultrasonore.

Pour ce faire, le signal électrique fourni au dispositif émetteur ultrasonore est adapté ou modifié par modulation de la phase du signal. En perturbant la phase du signal électrique, on peut avantageusement diminuer les harmoniques contenus dans ce signal et ainsi minimiser les rayonnements électromagnétiques (EM). Cette modulation peut être avantageusement mise en oeuvre dans un système ou dispositif émetteur existant, sans que cela nécessite des dispositifs supplémentaires et/ou des modifications structurelles du système ou dispositif existant. La complexité et les coût de mise en oeuvre du procédé peuvent donc être limités.

La limitation des émissions électromagnétiques peut être atteinte dans les divers modes de fonctionnement du dispositif émetteur ultrasonore. Des exemples de différents modes de fonctionnement d'un dispositif émetteur sous la forme d'une sonde à ultrasons peuvent comprendre un mode B (c'est-à-dire un mode brightness/luminosité), un mode Doppler ou un mode ShearWave(r) (c'est-à-dire un mode d'élastographie par ondes de cisaillement). En d'autres termes, il n'est pas nécessaire de changer ou de modifier le mode de fonctionnement spécifique d'un dispositif émetteur ultrasonore pour obtenir les réductions d'émissions électromagnétiques selon le concept de la présente divulgation. De telles réductions d'émissions électromagnétiques peuvent ainsi être atteintes indépendamment du mode de fonctionnement sélectionné, c'est-à-dire quel que soit le mode de fonctionnement mis en oeuvre par le dispositif.

Le procédé selon la divulgation peut comporter d'autres caractéristiques qui peuvent être prises séparément ou en combinaison, notamment parmi les modes de réalisation qui suivent qui sont présentés à titre d'illustration uniquement et peuvent être combinés ou associés sauf stipulation contraire.

Selon un exemple, la modulation de la phase du signal est configurée pour causer un élargissement du spectre fréquentiel du signal électrique.

Selon un exemple, la modulation de la phase du signal électrique est déterministe au cours du temps.

Selon un exemple, la modulation de la phase du signal électrique est aléatoire au cours du temps.

Selon un exemple, la modulation de la phase du signal électrique comprend :

- introduction, dans le signal électrique, d'au moins un délai correspondant à une période pendant laquelle la tension du signal électrique est maintenue à une valeur constante.

Selon un exemple, ledit au moins un délai est strictement inférieur à une demi-période du signal électrique.

Selon un exemple, au moins 4 délais sont introduits dans le signal électrique.

Selon un exemple, le signal électrique comprend une pluralité de séquences successives comprenant chacune au moins un cycle ondulatoire du signal électrique, un délai séparant chaque paire de séquences consécutives, le signal électrique généré au cours desdites séquences étant en phase.

Selon un exemple, la modulation de la phase du signal électrique comprend :

- un décalage angulaire de la phase du signal électrique dans au moins un cycle du signal électrique.

Selon un exemple, le décalage angulaire est configuré pour causer une inversion de la phase du signal électrique.

Selon un exemple, un décalage angulaire est introduit à chaque demi-période du signal électrique.

Selon un exemple, le décalage angulaires introduit dans le signal électrique croît, ou décroît, progressivement au cours du temps. Selon un exemple, le signal électrique comprend au moins une première période (ou première phase) au cours de laquelle le décalage angulaire introduit dans le signe électrique croît progressivement, et au moins une deuxième période (ou deuxième phase) au cours de laquelle le décalage angulaire introduit dans le signe électrique décroît progressivement.

Selon un exemple particulier, le signal électrique modifié par ladite modulation comprend au moins l'une quelconque parmi :

- au moins une première phase au cours de laquelle le décalage angulaire introduit dans le signal électrique croît progressivement ; et
- au moins une deuxième phase au cours de laquelle le décalage angulaire introduit dans le signal électrique décroît progressivement.

Selon un exemple, la modulation de la phase du signal électrique comprend une variation de la période des cycles du signal électrique au cours du temps.

Selon un exemple, la modulation de la phase est configurée pour que le signal électrique délivre une énergie électrique au moins égale à une énergie théorique qui serait délivrée par le signal électrique si sa phase n'était pas perturbée par ladite modulation de phase.

Selon un exemple, la modulation de la phase est réalisée tout en conservant une même fréquence sur chaque cycle du signal électrique.

Selon un exemple, les ondes ultrasonores sont des ondes de compression engendrant des ondes de cisaillement dans un milieu.

Selon un exemple, ledit procédé est appliqué à l'imagerie médicale par ultrasons.

Selon un autre aspect, la présente divulgation peut impliquer un programme d'ordinateur comprenant des instructions qui, lorsque le programme est exécuté par un ordinateur, cause la mise en oeuvre du procédé selon le premier aspect. En particulier, les différentes étapes du procédé selon le premier aspect peuvent être définies par des instructions de programmes d'ordinateurs.

Un tel programme d'ordinateur peut utiliser n'importe quel langage de programmation ou équivalent, et il peut se trouver sous la forme d'un code source, d'un code objet, ou d'un code intermédiaire entre un code source et un code objet, tel que dans une forme partiellement compilée, ou sous n'importe quelle autre forme souhaitable.

Selon un autre aspect, la présente divulgation concerne un support d'enregistrement (ou support d'informations), lisible par un ordinateur (ou un processeur), sur lequel est enregistré un programme d'ordinateur selon le ce même aspect de la présente divulgation.

D'une part, le support d'enregistrement peut être n'importe quel entité ou dispositif capable de stocker le programme, telle qu'au moins une mémoire volatile et/ou non volatile. Par exemple, le support peut comporter un moyen de stockage, tel qu'une mémoire non-volatile réinscriptible, une mémoire ROM, un CD-ROM ou une mémoire ROM de type circuit microélectronique, ou encore un moyen d'enregistrement magnétique ou un disque dur. Cette mémoire peut par exemple comprendre une mémoire de carte graphique (ou carte vidéo), ce type de mémoire étant notamment apte à traiter des données d'image (ou données vidéo).

D'autre part, ce support d'enregistrement peut également être un support transmissible tel qu'un signal électrique ou optique, un tel signal pouvant être acheminé via un câble électrique ou optique, par radio classique ou hertzienne ou par faisceau laser autodirigé ou par d'autres moyens. Le programme d'ordinateur selon la présente divulgation peut être en particulier téléchargé grâce à un réseau filaire ou non, de type local ou non (Bluetooth® par exemple, Wi-Fi, Ethernet, Internet, 4G, 5G ou autres).

Alternativement, le support d'enregistrement peut être un circuit intégré dans lequel le programme d'ordinateur est incorporé, le circuit intégré étant adapté pour exécuter ou pour être utilisé dans l'exécution du procédé en question.

Selon un autre aspect, la présente divulgation concerne un dispositif de traitement (ou dispositif de contrôle) d'un signal électrique pour un dispositif émetteur ultrasonore, ce dispositif étant configuré pour mettre en oeuvre le procédé du premier aspect de la présente divulgation.

Selon un exemple, le dispositif de traitement comprend une mémoire associée à un processeur, cette mémoire comprenant un programme d'ordinateur selon la présente divulgation.

Selon un exemple, la présente divulgation vise un dispositif de traitement d'un signal électrique pour un dispositif émetteur ultrasonore, ledit dispositif de traitement comprenant :

- un module de génération configuré pour générer un signal électrique définissant des ondes ultrasonores, la périodicité du signal électrique étant modifiée par une modulation de la phase du signal ; et
- un module de fourniture configuré pour fournir le signal électrique au dispositif émetteur ultrasonore pour causer l'émission des ondes ultrasonores.

Le dispositif de traitement peut avoir des fonctionnalités qui correspondent aux étapes (ou opérations) du procédé selon la présente divulgation. En particulier, les différents modes de réalisation mentionnés dans la présente divulgation en relation avec le procédé de la présente divulgation ainsi que les avantages associés peuvent s'appliquer de façon analogue au dispositif de traitement (et réciproquement).

Les caractéristiques et avantages de la divulgation apparaitront plus précisément à la lecture de la description qui va suivre, donnée uniquement à titre d'exemple non limitatif, et faite en référence aux figures annexées. Notamment, les exemples illustrés dans les figures peuvent être combinés entre eux, sauf incohérence notoire.

**Brève description des figures**

D'autres caractéristiques et avantages de la présente divulgation ressortiront de la description des exemples de réalisation non limitatifs de la présente divulgation exposés ci-après, en référence aux figures 1 à 14 annexées, sur lesquelles :

[Fig. 1] représente schématiquement un signal électrique, de l'art antérieur, sans modulation de phase, pour piloter un dispositif émetteur ultrasonore selon un exemple ;

[Fig. 2] représente schématiquement le spectre fréquentiel du signal électrique de la figure 1 sans modulation de phase selon un exemple ;

[Fig. 3] représente schématiquement un système ultrasonore, comprenant un dispositif de traitement et un dispositif émetteur ultrasonore, selon des exemples de réalisation de la présente divulgation ;

[Fig. 4] représente schématiquement le système ultrasonore de la figure 3, selon des exemples de réalisation de la présente divulgation ;

[Fig. 5] représente schématiquement le dispositif de traitement de la figure 3, selon des exemples de réalisation de la présente divulgation ;

[Fig. 6] représente schématiquement sous forme d'un diagramme les étapes d'un procédé de traitement mis en oeuvre par un dispositif de traitement, selon des exemples de réalisation de la présente divulgation ;

[Fig. 7] représente schématiquement la modulation d'un signal électrique au cours du procédé de traitement de la figure 6, selon des exemples de réalisation de la présente divulgation ;

[Fig. 8] représente schématiquement le spectre fréquentiel du signal électrique de la figure 7, selon des exemples de réalisation de la présente divulgation ;

[Fig. 9] représente schématiquement la modulation d'un signal électrique au cours du procédé de traitement de la figure 6, selon des exemples de réalisation de la présente divulgation ;

[Fig. 10] représente schématiquement le spectre fréquentiel du signal électrique de la figure 9, selon des exemples de réalisation de la présente divulgation ;

[Fig. 11] représente schématiquement la modulation d'un signal électrique au cours du procédé de traitement de la figure 6, selon des exemples de réalisation de la présente divulgation ;

[Fig. 12] représente schématiquement le spectre fréquentiel du signal électrique de la figure 11, selon des exemples de réalisation de la présente divulgation ;

[Fig. 13] représente schématiquement la modulation d'un signal électrique au cours du procédé de traitement de la figure 6, selon des exemples de réalisation de la présente divulgation ; et

[Fig. 14] représente schématiquement le spectre fréquentiel du signal électrique de la figure 13, selon des exemples de réalisation de la présente divulgation.

## Description des modes de réalisation

La présente divulgation porte sur des procédés et dispositifs pour le traitement (ou contrôle) d'un signal électrique pour un dispositif émetteur ultrasonore.

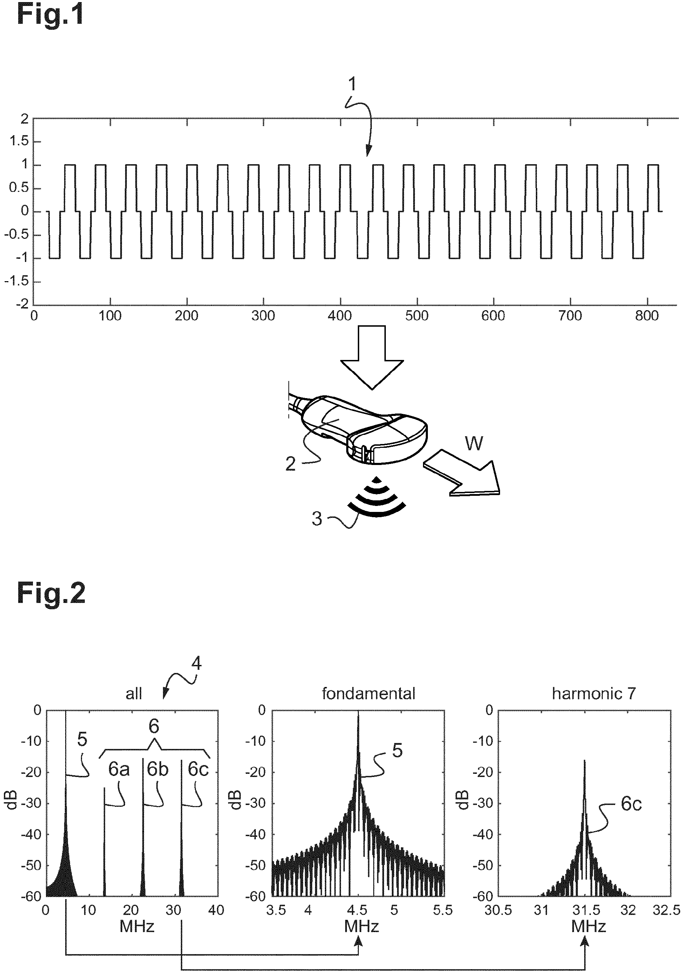

Comme illustré en figure 1 dans un exemple, un dispositif émetteur ultrasonore 2 est configuré pour émettre des ondes ultrasonores W en réponse à un ou des signaux électriques 1. Pour ce faire, un système de pilotage (non représenté) peut être configuré pour fournir ces signaux électriques 1 au dispositif émetteur ultrasonore 2. Ce système de pilotage peut en particulier comprendre un ou des pulseurs électroniques (dits plus simplement « pulseurs ») ou des amplificateurs linéaires, configurés pour générer le ou les signaux électriques 1 destinés à piloter le dispositif émetteur ultrasonore 2.

Le dispositif émetteur ultrasonore 2 peut comprendre un ou des transducteurs ultrasonores, chacun d'eux étant piloté par un signal électrique 1 délivré par le système de pilotage. La transmission de ces signaux électriques 1 peut être assurée par une chaîne de transmission depuis les pulseurs vers les transducteurs du dispositif émetteur 1.

Il a été constaté que la génération d'ondes ultrasonores W par un tel dispositif émetteur ultrasonore 2 engendre des émissions électromagnétiques (EM) 3 qu'il peut être souhaitable de minimiser, par exemple dans leur rayonnement, leurs directions, leurs intensités. Dans certains cas, il est souhaitable de limiter le rayonnement EM 3 d'un tel dispositif émetteur tout en s'assurant qu'il présente de bonnes performances, par exemple en termes de réponse spectrale et/ou d'énergie (ou puissance) acoustique délivrée. Il peut en particulier être souhaitable de limiter le niveau des émissions EM 3 avec un minimum d'impact sur les performances de fonctionnement souhaitées du dispositif émetteur ultrasonore 2.

Comme illustré en figure 2, le spectre fréquentiel 4 du signal électrique 1 fourni au dispositif émetteur ultrasonore 2 comprend généralement au moins un pic principal à une fréquence fondamentale 5 (délivrant une quantité importante d'énergie électrique) et des pics secondaires 6 à des fréquences harmoniques, à savoir des pics 6a, 6b et 6c dans cet exemple. Ces fréquences harmoniques correspondent à des multiples de la fréquence fondamentale. La génération de tels harmoniques résulte du caractère périodique du signal électrique ondulatoire 1.

Ces harmoniques 6 sont en dehors de la bande passante des transducteurs du dispositif émetteur ultrasonore 2 et ne sont pas converties en énergie acoustique mais dissipés sous forme de chaleur thermique, ce qui limite le rendement énergétique du dispositif. L'émission de telles harmoniques peut en outre conduire à des rayonnements électromagnétiques excessifs, ce qui peut poser problème notamment lorsqu'une limite maximale de ces rayonnements doit être respectée (par exemple conformément à une réglementation imposée pour obtenir une certification du système).

Pour limiter les harmoniques et éviter les rayonnements électromagnétiques trop importants, il est connu d'utiliser des pulseurs, ou amplificateurs, linéaires capables de générer des signaux sinusoïdaux, mais cette technique est particulièrement coûteuse et présente une efficacité moins ou peu adaptée, en particulier au domaine médical, notamment en raison du fait que les ondes ainsi générées sont limitées en puissance et produisent beaucoup de chaleur.

Les contraintes et/ou problèmes mentionnés précédemment imposent donc de réaliser des compromis ou de limiter certaines caractéristiques d'un tel dispositif émetteur ultrasonore afin de garantir de bonnes performances et un vieillissement acceptable, voire performant selon les exigences du marché, de l'ensemble. Ces contraintes et/ou problèmes peuvent en outre rendre difficile la qualification d'un tel dispositif émetteur d'ondes selon des normes en vigueur.

La présente divulgation se propose de répondre aux problèmes et contraintes précédemment décrits en traitant le signal électrique fourni à un dispositif émetteur ultrasonore afin de limiter le niveau des harmoniques contenus dans le signal électrique, tout en préservant le maximum souhaité d'énergie acoustique (ou le niveau du signal) à la fréquence fondamentale, de façon à garantir de bonnes performances du dispositif émetteur ultrasonore.

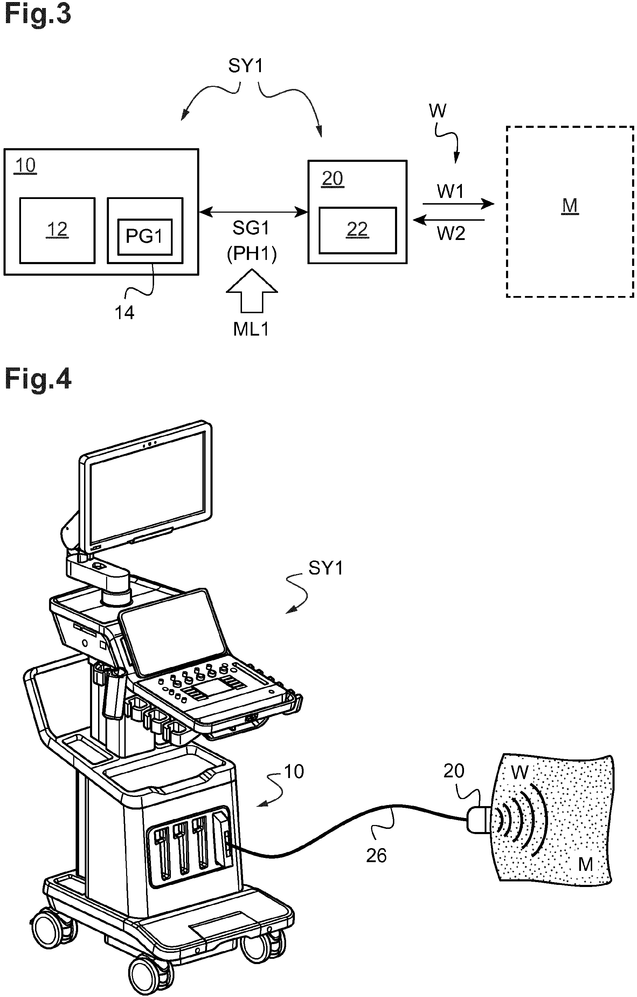

Des procédés et dispositifs de traitement (ou de contrôle) d'un signal électrique pour un dispositif émetteur ultrasonore vont à présent être décrits selon des modes particuliers de réalisation de la divulgation en référence conjointement aux figures 3 à 14. Sauf indications contraires, les éléments communs ou analogues à plusieurs figures portent les mêmes signes de référence et présentent des caractéristiques identiques ou analogues, de sorte que ces éléments communs ne sont généralement pas à nouveau décrits, par souci de concision.

Les termes « premier(s) » (ou première(s)), « deuxième(s) », etc.) sont utilisés dans ce document par convention arbitraire pour permettre d'identifier et de distinguer différents éléments (tels que des opérations, des dispositifs, etc.) mis en oeuvre dans les modes de réalisation décrits ci-après.

Comme précédemment indiqué, la présente divulgation vise notamment un procédé de traitement mis en oeuvre par un dispositif émetteur ultrasonore, ainsi qu'un tel dispositif. La figure 3 représente schématiquement un système ultrasonore SY1 comprenant un dispositif de traitement (ou dispositif de contrôle) 10 et un dispositif émetteur ultrasonore 20 (dit aussi dispositif d'émission ultrasonore, ou dispositif d'émission d'ondes ultrasonores, ou dispositif transducteur ultrasonore) selon des exemples de réalisation de la présente divulgation.

Plus spécifiquement, le dispositif de traitement 10 est configuré pour contrôler (ou piloter) le dispositif émetteur ultrasonore 20. A cet effet, le dispositif de traitement 10 génère un ou des signaux électriques SG1 qu'il transmet au dispositif ultrasonore 20 pour causer l'émission d'ondes ultrasonores W1. A des fins de simplification de l'exposé de la présente divulgation, on considère par la suite que le dispositif de traitement 10 génère et transmet un signal électrique SG1 au dispositif ultrasonore 20, étant entendu que ce signal électrique SG1 peut comprendre une pluralité de signaux électriques. Le signal électrique SG1 ainsi émis permet de piloter les ondes ultrasonores W1 émises par le dispositif émetteur 20.

Le dispositif émetteur ultrasonore 20, dit aussi par la suite dispositif ultrasonore, est configuré pour émettre, et éventuellement aussi recevoir, des ondes W2. Ce dispositif ultrasonore 20 peut par exemple prendre la forme d'une sonde à ultrasons (par exemple une sonde échographique). La nature de ces ondes ultrasonores W dépend de la configuration du dispositif ultrasonore 20, au vu notamment de l'utilisation qui en est faite.

Selon un exemple, le dispositif de traitement 10 et le dispositif ultrasonore 20 sont des dispositifs distincts. Selon des variantes, tout ou partie du dispositif de traitement 10 peut être mis en oeuvre dans le dispositif ultrasonore 20.

De façon générale, le système ultrasonore SY1, et plus précisément le dispositif de traitement 10 et le dispositif émetteur ultrasonore 20 peuvent être stationnaires ou mobiles.

Selon un exemple, le dispositif de traitement 10 et le dispositif ultrasonore 20 forme un seul et même dispositif, stationnaire ou mobile selon le cas.

Par exemple, le dispositif ultrasonore 20 peut être connecté au dispositif de traitement 10 par un câble ou peut communiquer sans fil avec lui. Dans ce dernier cas, le dispositif ultrasonore 20 peut par exemple comprendre une batterie et recevoir des signaux de communication du dispositif de traitement 10, ces signaux représentant le signal électrique SG1 (par exemple les fréquences de pilotage et/ou toute information comprise dans le signal électrique). Le dispositif ultrasonore 20 peut alors reproduire le signal électrique SG1 en interne à partir des signaux de communication reçus.

Le dispositif ultrasonore 20 peut par exemple être un dispositif émetteur conventionnel d'ondes ultrasonores, ce dispositif étant piloté au moyen du dispositif de traitement 10 selon un procédé de traitement conforme à la présente divulgation.

Comme illustré en figure 3, le dispositif de traitement 10 comprend dans cet exemple un processeur 12 et une mémoire 14. Le dispositif de traitement 10 est configuré pour piloter le dispositif ultrasonore 20 par la génération et l'envoi d'un signal électrique SG1. Une fois généré, ce signal électrique SG1 est ainsi transmis au dispositif ultrasonore 20 pour causer l'émission, par ledit dispositif ultrasonore 20, d'ondes ultrasonores W1 en direction et/ou dans un milieu M. Le signal électrique SG1 ainsi généré est représentatif des (ou définit les) ondes ultrasonores W1 projetées dans le milieu M. À cette fin, l'unité de traitement 10 peut par exemple être ou comprendre au moins un pulseur électronique, dit aussi « pulseur » apte à générer le signal électrique.

Le signal électrique SG1 est un signal ondulatoire (ou alternatif) qui est traité par le dispositif de traitement 10 pour piloter le dispositif ultrasonore 20 de sorte à obtenir de bonnes performances du dispositif ultrasonore 20 tout en minimisant ses émissions EM. Comme décrit par la suite, le dispositif de traitement 10 est configuré pour modifier la périodicité du signal électrique SG1 par une modulation ML1 de la phase PH1 (figure 3) du signal électrique SG1. Le dispositif de traitement 10 peut en outre réaliser divers traitements lors de la génération du signal électrique SG1 (amplification, filtration, numérisation, conditionnement du signal, etc.).

Dans des exemples, le processeur 12 et la mémoire 13 peuvent être incorporés dans le dispositif de traitement 10 illustré en figure 3 ou peuvent être incorporés dans un ordinateur ou un dispositif informatique lié de manière communicante à celui-ci.

La mémoire 14 peut stoker sous forme d'un programme d'ordinateur PG1 des instructions définissant les étapes des procédés décrits dans la présente divulgation. A ce titre, la mémoire 14 constitue un support d'enregistrement (ou support d'informations) conforme à des modes particuliers de réalisation, lisible par le dispositif de traitement 10, et sur lequel est enregistré un programme d'ordinateur PG1 conforme à des modes particuliers de réalisation. Ce programme d'ordinateur PG1 comporte des instructions pour l'exécution des étapes d'un procédé de traitement dont des modes particuliers de réalisation sont décrits dans la présente divulgation. Le processeur 12 est ainsi configuré pour exécuter les instructions du programme d'ordinateur PG1 afin de réaliser des étapes du procédé de traitement.

Selon la configuration et le type de dispositif informatique considéré, la mémoire 14 peut être volatile (telle que la RAM), non volatile (telle que de la mémoire ROM, flash, EEPROM, etc. ou tout autre dispositif de stockage et/ou support lisible par ordinateur comme décrit ci-après) ou une combinaison des deux. La mémoire 14 peut, par exemple, être gérée en mode DMA (pour « Direct Memory Access » en anglais). La mémoire 14 utilisée par l'unité de traitement 10 peut par exemple comprendre tout ou partie d'une mémoire de carte graphique (ou carte vidéo), ce type de mémoire étant notamment apte à traiter et/ou envoyer des données d'image pouvant être utilisées pour afficher une ou des images sur un écran (ou une unité) d'affichage.

Le dispositif de traitement 10 peut prendre la forme de divers moyens informatiques appropriés (tels par exemple station de travail, ordinateur, serveur, etc.) comprenant tout ou partie des éléments décrits ci-dessus ainsi qu'éventuellement d'autres éléments non mentionnés.

Comme illustré en figure 3, le dispositif ultrasonore 20 peut comprendre par exemple un ou plusieurs transducteurs (dit aussi éléments transducteurs) notés 22, chacun étant configuré pour convertir un signal électrique SG1 fourni par le dispositif de traitement 10 en ondes ultrasonores W (et éventuellement aussi réciproquement). Les transducteurs 22 peuvent ainsi être configurés pour émettre des ondes (ou impulsions ultrasonores, ou faisceaux ultrasonores) W1 dans le milieu M, ce qui correspond à une opération d'émission. Ces éléments transducteurs peuvent être disposés de façon quelconque, par exemple selon une ligne de transducteurs, une matrice, ou selon un réseau de transducteurs ou toute autre configuration appropriée.

A noter que les transducteurs 22 peuvent éventuellement aussi être configurés pour recevoir des signaux ultrasonores W2 du milieu M dans une opération de réception, par exemple en réponse aux ondes W1 transmises, bien que des variantes selon lesquelles le dispositif ultrasonore 20 fonctionne uniquement en émission sont également possibles.

Le système ultrasonore SY1 (figure 3) peut être un système d'imagerie ultrasonore, par exemple dans le domaine médical. Les images ultrasonores générées par le système peuvent être soit analysées en temps réel, par exemple par un utilisateur ou un algorithme, et/ou un module d'intelligence artificielle, soit analysées ultérieurement et/ou dans un autre lieu que celui dans lequel se trouve le système SY1.

Le système SY1 peut être un système médical à ultrasons. De même, le dispositif émetteur 20 peut être une sonde médicale à ultrasons.

Par exemple, le système SY1 peut être associé à une sonde à ultrasons 20, afin d'étudier un milieu M (figure 3), notamment pour collecter des données ultrasonores d'un tel milieu M. Le milieu M ainsi observé peut être de diverses natures selon le cas. Il peut s'agir par exemple de métaux, de tissus d'êtres vivants, en particulier des tissus humains ou tissus d'animaux. L'observation d'un milieu M comprenant une ou des structures minérales par exemple est également possible (graviers, volcan, cartographie d'un sol, par exemple de fonds marins, etc.).

Le système ultrasonore SY1 peut être configuré pour diverses applications, notamment dans les domaines de l'acoustique, de l'étude des matériaux, de l'imagerie médicale et/ou de la biomédecine.

La figure 4 représente schématiquement un exemple de réalisation non limitatif du système SY1 tel que précédemment décrit en référence à la figure 3, à savoir en l'espèce un système d'imagerie ultrasonore. Dans cet exemple, l'unité de traitement 10 est compris dans le corps d'une station de commande, cette dernière étant en outre munie d'une interface de commande et d'un dispositif d'affichage. Le dispositif ultrasonore 20 prend la forme d'une sonde ultrasonore, reliée dans cet exemple de façon communicante avec le dispositif de traitement 10 via un câble de connexion 26.

Le système ultrasonore SY1 peut être configuré pour réaliser une image ultrasonore de diverses natures, par exemple une image B-mode du milieu M (image en mode B affichée en niveaux de gris), une image dite Doppler illustrant les mouvements de fluides dans le milieu observé, et/ou une image montrant une caractéristique mécanique du milieu (par exemple des données d'image d'élastographie obtenues à l'aide d'ondes de cisaillement (« ShearWave™ Elastography »)). Selon un exemple, le signal électrique SG1 transmis par le dispositif de traitement 10 cause ainsi l'émission par le dispositif ultrasonore 20 d'ondes ultrasonores de compression engendrant des ondes de cisaillement dans un milieu M.

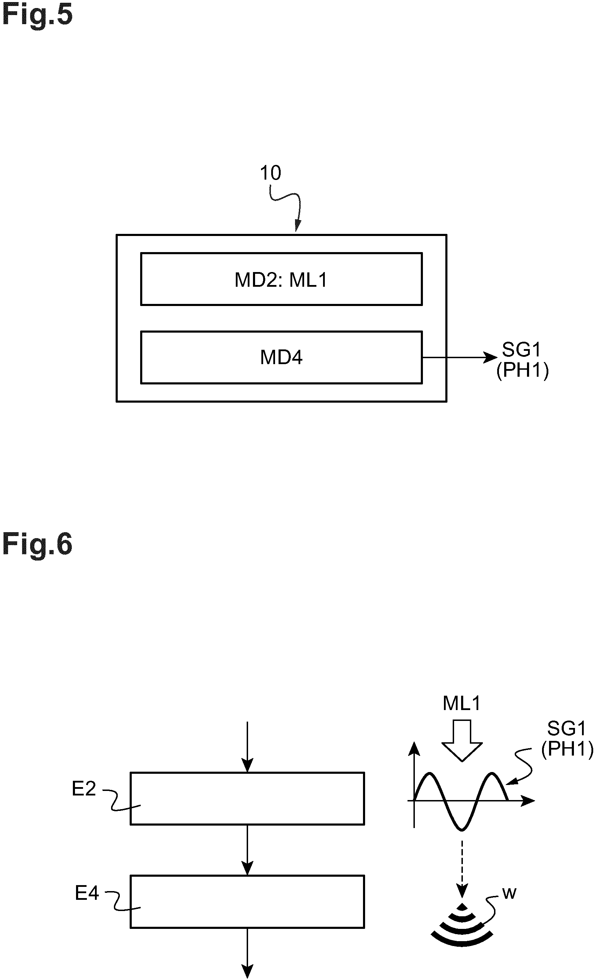

Comme représenté en figure 5 selon un mode de réalisation, le processeur 12 du dispositif de traitement 10, piloté par le programme d'ordinateur PG1 (figure 3), peut mettre en oeuvre un module de génération MD2 et un module de fourniture MD4.

Plus spécifiquement, le module de génération MD2 peut être configuré pour générer un signal électrique SG1 définissant des ondes ultrasonores W, la périodicité du signal électrique SG1 étant modifiée (ou adapté) par une modulation ML1 de la phase PH1 du signal SG1.

Le module de fourniture (ou module de transmission) MD4 peut être configuré pour fournir le signal électrique SG1 au dispositif émetteur ultrasonore 20 pour causer l'émission des ondes ultrasonores W.

La configuration et le fonctionnement des modules MD2-MD4 du dispositif de traitement 10 apparaîtront plus précisément dans les exemples de réalisation décrits ci-après en référence aux figures. Les modules MD2-MD4 tels que représentés en figure 5 ne représentent qu'un exemple de mise en oeuvre non limitatif de l'invention.

De manière générale, pour chaque étape du procédé de traitement de la présente divulgation, le dispositif de traitement de la divulgation peut comprendre un module correspondant configuré pour réaliser ladite étape (et réciproquement).

Des modes de réalisation du procédé de traitement de la divulgation sont à présent décrits en référence aux figures 6 à 14. Dans ces exemples, le procédé de traitement est mis en oeuvre par le système ultrasonore SY1 (figures 3, 4 et 5), et plus précisément par le dispositif de traitement 10. Pour ce faire, le dispositif de traitement 10, coopérant avec le dispositif ultrasonore 20, peut exécuter le programme d'ordinateur PG1.

Au cours d'une étape E2 de traitement (figure 6), le dispositif de traitement 10 génère un signal électrique SG1 définissant des ondes ultrasonores W, la périodicité du signal électrique SG1 étant modifiée, adaptée, ou contrôlée par une modulation ML1 de la phase PH1 du signal SG1.

Le signal électrique SG1 est un signal ondulatoire (ou alternatif) destiné à piloter le dispositif ultrasonore 20. En réalisant la modulation ML1, la phase PH1 (ou la périodicité) du signal SG1 se trouve perturbée. Cette modulation PH1 peut avoir pour effet que le signal électrique SG1 n'est pas totalement périodique ou présente une périodicité modifiée par rapport à un cas théorique où une telle modulation ML1 ne serait pas appliquée sur le signal SG1.

Selon un exemple, la phase PH1 est la phase instantanée. Comme le comprend l'homme du métier, la phase instantanée du signal électrique SG1 diffère de la phase à l'origine. Par exemple, pour un signal sinusoïdal SG1, ce signal peut se définir comme suit :

$$[\text{Math. 1}]\ SG1 = \sin(2 \cdot \pi \cdot f0 \cdot t + \varphi)$$

où $\varphi$ est la phase à l'origine et où $(2*\pi*f0*t + \varphi)$ est la phase instantanée à l'instant t.

La modulation ML1, réalisables de diverses manières comme décrit ci-après dans des exemples, peut en particulier être configurée pour causer un élargissement du spectre fréquentiel du signal électrique SG1 (et donc des ondes ultrasonores W émises en sortie). Cet élargissement permet de diminuer les harmoniques en fréquence contenus dans le signal électrique SG1 et ainsi de réduire les émissions EM du dispositif ultrasonore 20, ou plus généralement du système ultrasonore SY1.

Au cours d'une étape E4 de fourniture ou de transmission (figure 6), le dispositif de traitement 10 fournit le signal électrique SG1 au dispositif ultrasonore 20 pour causer l'émission des ondes ultrasonores W1.

Le signal électrique SG1 peut comprendre des cycles périodiques. La forme d'onde du signal peut varier selon les cas. Le nombre de cycles au cours desquels est transmis le signal électrique SG1 peut également être adapté selon le cas.

Selon un exemple, le signal électrique SG1 est transmis pendant au moins plusieurs dizaines de cycles du signal, de sorte à causer une émission continue d'ondes ultrasonores W1 vers un milieu M (par exemple au cours d'une émission ou « push » d'une durée de 100 $\mu$s environ). Les ondes ultrasonores W1 ainsi émises sont par exemple des ondes de compression engendrant des ondes de cisaillement dans le milieu M considéré (selon un mode d'élastographie par ondes de cisaillement). Par nature, l'émission continue de ce type d'ondes a tendance à générer un rayonnement EM important, ce qu'il peut être souhaitable d'éviter.

Selon un exemple, la modulation ML1 de la phase PH1 du signal électrique SG1 est déterministe au cours du temps. Autrement dit, la modulation PH1 est mise en oeuvre selon une configuration prédéfinie. En particulier, la sélection du ou des cycles du signal SG1 dans lesquels la phase PH1 est modulée, peut être réalisée de façon déterministe ou prédéfinie.

Selon un exemple, la modulation ML1 de la phase PH1 du signal électrique SG1 est aléatoire au cours du temps. En particulier, la sélection du ou des cycles du signal SG1 dans lesquels la phase PH1 est modulée, peut être réalisée de façon aléatoire.

La présente divulgation permet avantageusement de limiter les rayonnements EM émis par le dispositif émetteur ultrasonore 20 tout en assurant à ce dernier des performances satisfaisantes, en termes par exemple de réponse spectrale et/ou d'énergie (ou puissance) acoustique délivrée. On peut ainsi avantageusement limiter le niveau des émissions EM avec un minimum d'impact (voire aucun impact) sur les performances de fonctionnement souhaitées du dispositif émetteur ultrasonore 20.

Pour ce faire, le signal électrique fourni au dispositif émetteur ultrasonore 20 est adapté (ou traité) par modulation de la phase PH1 du signal SG1. En perturbant la phase PH1 du signal électrique SG1, on peut avantageusement diminuer les harmoniques contenus dans ce signal et ainsi minimiser les rayonnements EM. Cette modulation peut être avantageusement mise en oeuvre dans un système ou dispositif émetteur existant, sans que cela nécessite des dispositifs supplémentaires et/ou des modifications structurelles du système ou dispositif existant. La complexité et les coûts de mise en oeuvre du procédé s'en trouvent donc limités.

En particulier, on peut avantageusement concevoir un dispositif émetteur ultrasonore permettant des utilisations optimales, performant, tout en générant un minimum d'émission électromagnétique en fonctionnement et présentant une complexité de conception, de fabrication et de mise en oeuvre et d'utilisation limitée.

La limitation des émissions électromagnétiques peut être atteinte dans divers modes de fonctionnement du dispositif émetteur ultrasonore. Des exemples de différents modes de fonctionnement d'un dispositif émetteur sous la forme d'une sonde à ultrasons peuvent comprendre un mode B (c'est-à-dire un mode de luminosité), un mode Doppler ou un mode ShearWave(r) (c'est-à-dire un mode d'élastographie par ondes de cisaillement). En d'autres termes, il n'est pas nécessaire de changer ou de modifier le mode de fonctionnement spécifique d'un dispositif émetteur ultrasonore pour obtenir les réductions d'émissions EM, ceci pouvant être atteintes indépendamment de la sélection du mode de fonctionnement du dispositif.

Pour référence, les figures 1 et 2 représentent un signal électrique 1 de référence sans modulation ML1, équivalent au signal électrique SG1 qui serait théoriquement généré dans les exemples de la présente divulgation si aucun déphasage ML1 n'était appliqué conformément à la présente divulgation.

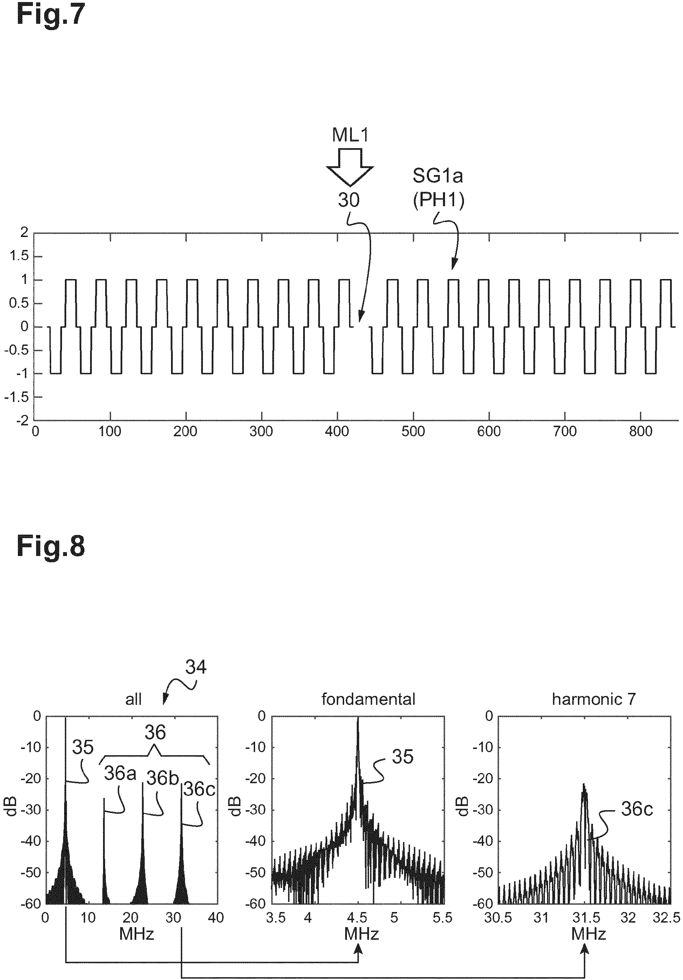

Des exemples de réalisation de la modulation ML1 du signal électrique SG1 mise en oeuvre par le dispositif de traitement 10 au cours de l'étape E2 de génération de la méthode de traitement sont à présent décrits en référence aux figures 7 à 14.

A noter qu'il est possible de mettre en oeuvre la modulation ML1 du signal électrique SG1 selon divers modes de réalisation, tels que ceux décrits ci-après, cette modulation ML1 pouvant combiner au moins deux modes de réalisation quelconques parmi ceux décrits ci-après en référence notamment aux figures 7-14. Autrement dit, il est possible d'appliquer de façon cumulative une pluralité des techniques de modulation décrites ci-après dans des exemples.

Selon un premier mode de réalisation illustré en figures 7 et 8, la modulation ML1 de la phase PH1 du signal électrique SG1, noté en l'espèce SG1a, est réalisée (étape E2, figure 6) par introduction (ou ajout), dans le signal électrique SG1a, d'au moins un délai (ou période d'attente) 30, correspondant à une période pendant laquelle la tension du signal électrique SG1a est maintenue à une valeur constante. A titre d'exemple, cette valeur constante est fixée à zéro (0 Volt), bien que des valeurs différentes soient possibles.

La figure 7 représente dans cet exemple le signal électrique SG1a modulé, où le temps est représenté selon l'axe des abscisses (en nombre de cycles d'horloge) et la tension du signal est représentée selon l'axe des ordonnées. Comme illustré, un seul délai 30 est introduit dans le signal électrique SG1 dans cet exemple, bien qu'il soit possible d'en introduire une pluralité.

L'introduction d'un ou plusieurs délais temporels 30 entraîne un découpage en N sous-émissions ultrasonores, plus courtes que si aucun délai temporel n'était appliqué, N étant un nombre entier au moins égal à 1. On peut ainsi obtenir un signal électrique SG1a périodique discontinu. Autrement dit, l'ajout du ou des délais 30 perturbe la périodicité du signal électrique SG1a.

Selon un exemple, N est égal à 2 ou plus, ce qui correspond à l'introduction d'une pluralité de délais 30 dans le signal électrique SG1a. L'ajout d'un délai 30 entre les sous-émissions ultrasonores permet avantageusement de minimiser les harmoniques contenues dans le signal électrique SG1a (et donc dans les ondes ultrasonores W en sortie) et ainsi de limiter le rayonnement EM causé par ces harmoniques.

Selon un exemple, la modulation ML1 est réalisée de sorte que N est au moins égal à 4 (N ≥ 4). On peut ainsi introduire des délais 30 entre des sous-émissions ultrasonores formant collectivement le signal électrique SG1a. Le répétition de ces délais 30 permet avantageusement de produire un signal électrique SG1a, et donc un onde ultrasonore W, sur une relativement longue durée tout en minimisant efficacement les rayonnements électromagnétiques. En particulier, la répétition des délais 30 au cours du temps (par exemple 10, 20, 30 ou 40 délais 30) permet d'élargir le spectre spectral du signal et donc de causer une redistribution de l'énergie électrique, ce qui conduit à une réduction du pic de la densité spectrale des émissions électromagnétiques.

A titre d'exemple, on peut avantageusement générer des ondes de compression sous la forme d'émissions continues ultrasonores, dites « pushs », c'est-à-dire des cycles de signaux électriques SG1 qui sont entrecoupés de délais 30 (par exemple 4 délais ou plus) et permettent la création d'ondes de cisaillement dans le milieu d'intérêt tout en limitant les rayonnements électromagnétiques susceptibles d'être émis par la sonde 20.

Selon un exemple, la durée du ou des délai 30 introduits dans le signal électrique SG1 est strictement inférieure à une demi-période (voire à un tier ou un quart de la période) du signal électrique SG1. On peut ainsi minimiser les temps d'attente pendant les délais 30, ce qui permet d'accélérer la génération des ondes ultrasonores W, tout en limitant les rayonnements électromagnétiques du fait que l'on perturbe efficacement la périodicité du signal. Ce mode de réalisation est en particulier adapté pour générer des ondes de cisaillement, ce qui nécessite la génération d'un « push » relativement long du signal électrique SG1. Il devient alors critique de limiter au maximum la durée des délais 30 pour permettre l'exécution du procédé dans un temps restreint.

Selon un exemple particulier, le signal électrique SG1 généré en E2 (figure 6) comprend une pluralité de séquences successives, chaque séquence comprenant au moins un cycle ondulatoire (ou au moins une période) du signal électrique SG1 selon une fréquence donnée, un délai 30 étant appliqué entre (ou séparant) chaque paire de séquences consécutives. Selon un exemple, le signal électrique SG1 généré au cours de toutes ces séquences au cours du temps est en phase (voire est identique), ce qui permet avantageusement de limiter les perturbations ou interférences pouvant résulter entre les différentes séquences du signal électriques SG1. Selon un exemple, la fréquence du signal électrique SG1 dans chaque séquence est identique (autrement dit, la fréquence est constante hors des périodes de délai), ce qui permet notamment avantageusement d'utiliser un transducteur ayant une bande passante fréquentielle très étroite ou de répéter la même forme d'onde décalée dans le temps pour diminuer l'espace mémoire nécessaire au stockage des formes d'ondes émises.

Plus spécifiquement, comme illustré en figure 8, le spectre fréquentiel 34 du signal électrique SG1a comprend par exemple un pic principal 35 à une fréquence fondamentale et des pics secondaires 36a, 36b et 36c (notés collectivement 36) à des fréquences respectives d'harmoniques. La modulation ML1 par ajout d'un délai 30 dans cet exemple permet de réduire sensiblement les harmoniques 36, notamment l'harmonique 36c (multiple de 7 de la fréquence fondamentale), et ce tout en maintenant à un niveau élevé le pic 35 à la fréquence fondamentale. En particulier, la diminution des harmoniques 36 résulte d'un élargissement spectral causé par le délai temporel 30 qui perturbe la périodicité du signal SG1a.

Selon un exemple, le ou les délais 30 sont configurés pour être inférieurs à la période du signal électrique SG1a, c'est-à-dire inférieurs à la durée d'un cycle périodique du signal SG1a. De cette manière, on peut avantageusement conserver la densité d'énergie électrique délivrée par le signal SG1 malgré l'application de la modulation ML1.

A titre d'exemple, le signal électrique SG1a ainsi modulé produit une émission continue ultrasonore (ou « push ») de 100 $\mu$s environ selon une période de 0,5 $\mu$s. Le ou les délais 30 sont par exemple de l'ordre de 0,2 $\mu$s environ.

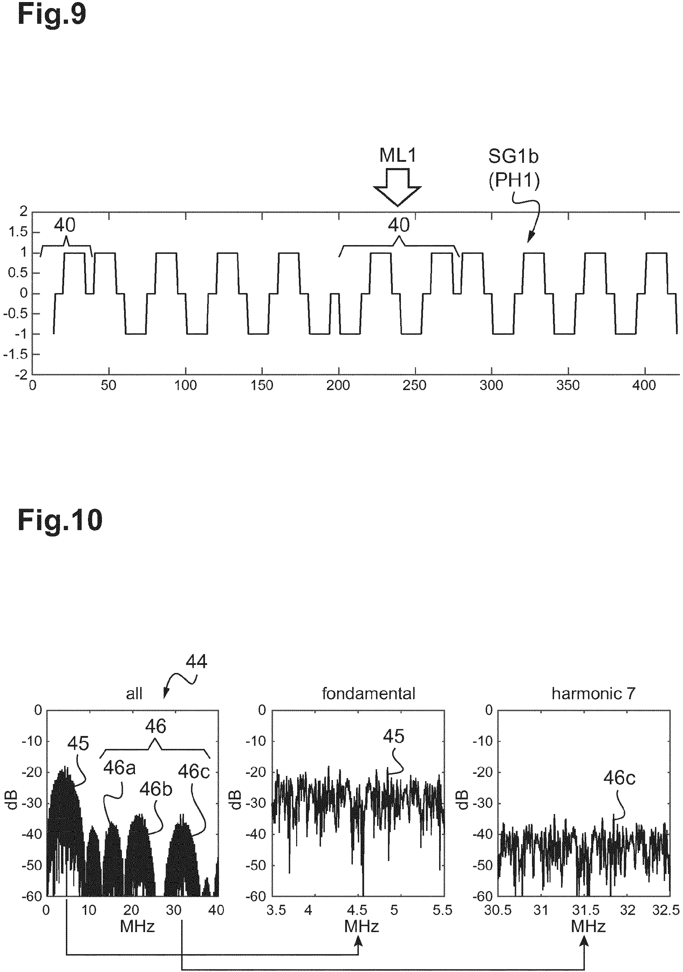

Selon un deuxième mode de réalisation illustré en figures 9 et 10, la modulation ML1 de la phase PH1 du signal électrique SG1, notée en l'espèce SG1b, est réalisée (étape E2, figure 6) par application (ou introduction) d'un décalage angulaire de la phase PH1 du signal électrique SG1b dans au moins un cycle dudit signal. Ce décalage angulaire 40 correspond à un déphasage du signal SG1 selon une valeur angulaire donnée. La valeur angulaire de ce décalage peut être adaptée selon le cas. A titre d'exemple, cette valeur angulaire peut être fixée à 30° ou 40°, bien que d'autres valeurs soient possibles.

Selon un exemple, un décalage angulaire 40 est appliqué sur un seul cycle de signal électrique SG1a. Selon un autre exemple, un tel décalage angulaire 40 est appliqué sur une pluralité de cycles du signal électrique SG1b. Le décalage 40 peut avoir une même valeur ou une valeur différente d'un cycle déphasé à un autre.

La figure 9 représente dans cet exemple le signal électrique SG1b modulé, où le temps est représenté selon l'axe des abscisses (en nombre de cycles d'horloge) et la tension du signal est représentée selon l'axe des ordonnées. Comme illustré dans cet exemple, un décalage angulaire 40 d'une valeur de 180° est appliqué au signal électrique SG1 dans certains cycles par rapport à d'autres cycles où le signal n'est pas déphasé.

L'application d'un ou plusieurs décalages angulaires 40 entraîne un déphasage entre les cycles du signal électrique SG1b, de sorte qu'au moins un cycle du signal est déphasé par rapport à au moins un autre cycle du signal. Dans ce cas, on peut ainsi obtenir un signal périodique continu dont la périodicité est perturbée ou modifiée par le ou les décalage angulaires 40.

Dans l'exemple représenté en figure 9, le décalage angulaire de la phase PH1 est fixé à une valeur de 180°, causant une inversion de signe du signal électrique dans le ou les cycles déphasés.

L'application d'un ou plusieurs décalages angulaires permet avantageusement de minimiser les harmoniques contenues dans le signal électrique SG1b (et donc dans les ondes ultrasonores W en sortie) et ainsi de limiter le rayonnement EM causé par ces harmoniques.

Plus spécifiquement, comme illustré en figure 10, le spectre fréquentiel 44 du signal électrique SG1b comprend par exemple un pic principal 45 à une fréquence fondamentale et des pics secondaires 46a, 46b et 46c (notés collectivement 46) à des fréquences respectives d'harmoniques. La modulation ML1 par décalage angulaire dans cet exemple permet de réduire sensiblement les harmoniques 46, notamment l'harmonique 46c (multiple de 7 de la fréquence fondamentale), et ce tout en maintenant à un niveau élevé le pic 45 à la fréquence fondamentale.

En particulier, la diminution des harmoniques 46 résulte d'un élargissement spectral causé par le décalage angulaire de la phase qui perturbe la périodicité du signal SG1b. Comme illustré, on obtient en effet une multiplication des pics en fréquence contenus dans le signal SG1b par rapport à si aucune modulation ML1 n'était appliquée, ce qui se traduit par une redistribution de l'énergie électrique dans le spectre fréquentiel du signal SG1b.

Selon un exemple, un décalage angulaire 40 de 180° est appliqué aléatoirement au signal électrique SG1b pour causer une inversion du signal SG1b dans au moins un cycle choisi aléatoirement. Un tel décalage angulaire aléatoire peut également être appliqué pour une valeur non nulle autre que 180°.

Selon un exemple, un décalage angulaire 40 de 180° est appliqué de façon déterministe au signal électrique SG1b pour causer une inversion du signal SG1b dans au moins un cycle choisi de façon prédéfinie. Un tel décalage angulaire déterministe peut également être appliqué pour une valeur non nulle autre que 180°.

Selon un exemple, des décalages angulaires 40 de différentes valeurs sont appliqués respectivement à une pluralité de cycles du signal électrique SG1b.

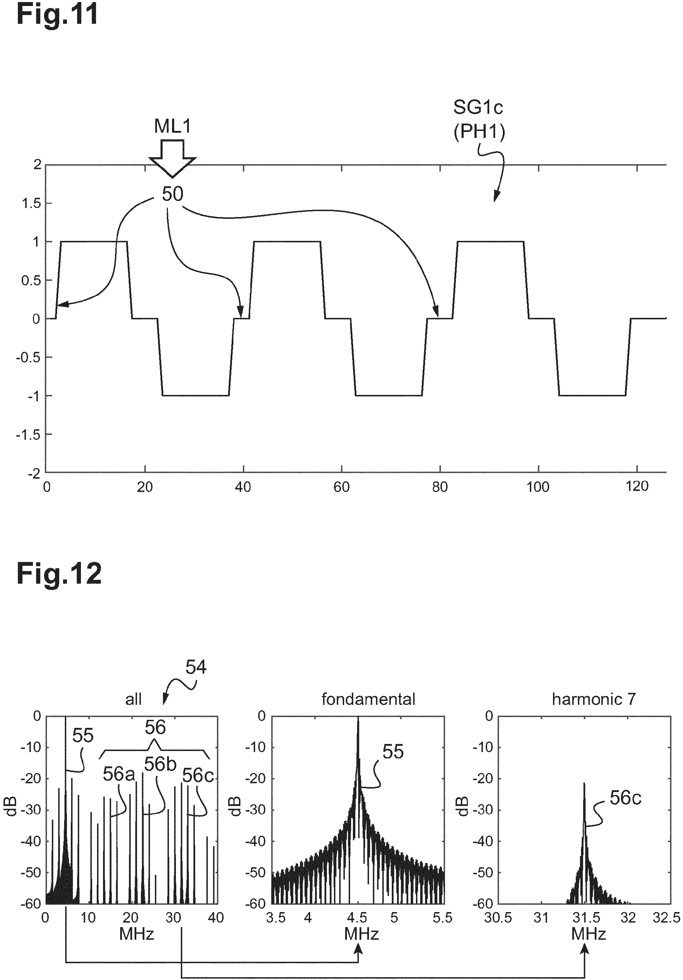

Les figures 11 et 12 représentent un exemple analogue à celui des figures 9-10, dans lequel la modulation ML1 de la phase PH1 du signal électrique SG1, notée en l'espèce SG1c, est réalisée (étape E2, figure 6) par application de décalages angulaires, notés 50 (figure 11), de 18°, 35° et 53° de la phase PH1 du signal électrique SG1. Cela équivaut à appliquer au signal SG1c des déphasages selon ces valeurs angulaires.

Plus spécifiquement, comme illustré en figure 12, le spectre fréquentiel 54 du signal électrique SG1c comprend par exemple un pic principal 55 à une fréquence fondamentale et des pics secondaires 56a, 56b et 56c (notés collectivement 56) à des fréquences respectives d'harmoniques. La modulation ML1 par décalage angulaire dans cet exemple permet de réduire sensiblement les harmoniques 56, notamment l'harmonique 56c (multiple de 7 de la fréquence fondamentale), et ce tout en maintenant à un niveau élevé le pic 55 à la fréquence fondamentale.

La diminution des harmoniques 56 résulte d'un élargissement spectral causé par le décalage angulaire de la phase qui perturbe la périodicité du signal SG1c. Comme illustré, on obtient une multiplication des pics en fréquence contenus dans le signal SG1c par rapport au cas où aucune modulation ML1 n'est appliquée, ce qui se traduit par une redistribution de l'énergie électrique dans le spectre fréquentiel du signal SG1c.

Selon un exemple, la modulation ML1 de la phase PH1 du signal électrique SG1, est réalisée (étape E2, figure 6) par application (ou introduction) d'un décalage angulaire 50 à chaque demi-période du signal SG1. Ce décalage angulaire 50 peut être de même valeur à chaque demi-période, ou varier selon le cas. En appliquant deux décalages angulaires 50 par période dans le signal électrique SG1c, on peut efficacement élargir le spectre fréquentiel de ce signal et ainsi minimiser le pic des émissions électromagnétiques tout en maintenant un niveau élevé d'énergie électrique délivrée par le signal électrique SG1.

Selon un exemple, les décalages angulaires 50 introduits dans le signal SG1 croissent, ou décroissent, progressivement au cours du temps. A titre d'exemple, le décalage angulaire introduit à chaque demi-période du signal SG1 croît, ou décroît, progressivement au cours du temps. On peut ainsi former par exemple des ondes de plus en plus rapprochées, ou distantes, dans le temps, ce qui permet avantageusement de répartir efficacement l'énergie électrique du signal électrique SG1 (élargissement spectrale), et donc de minimiser les émissions électromagnétiques. Une combinaison de phases de décalages angulaires croissants et décroissants est également possible. Ainsi, selon un exemple, le signal électrique SG1 comprend au moins une première période (ou première phase) au cours de laquelle le décalage angulaire introduit dans le signe électrique croît progressivement, et au moins une deuxième période (ou deuxième phase) au cours de laquelle le décalage angulaire introduit dans le signe électrique décroît progressivement. Le signal électrique SG1 peut en particulier comprendre une alternance de premières et deuxièmes phases dans lesquelles ces décalages angulaires augmentent et diminuent respectivement au cours du temps.

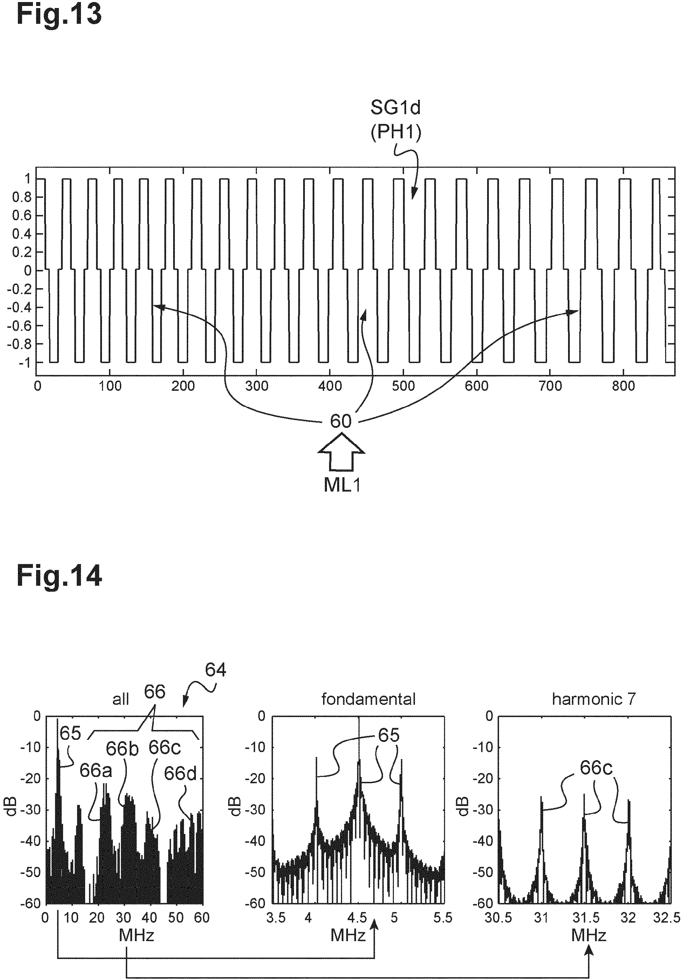

Selon un quatrième mode de réalisation illustré en figures 13 et 14, la modulation ML1 de la phase PH1 du signal électrique SG1, notée en l'espèce SG1d, est réalisée (étape E2, figure 6) par une variation 60 au cours du temps de la période des cycles du signal électrique SG1d.

Cette variation 60 de la période peut ainsi comprendre par exemple une augmentation et/ou une diminution de la période du signal électrique SG1c au cours du temps. La manière dont la période du signal SG1d varie au cours du temps peut être adaptée selon le cas.

Selon un exemple, une variation 60 de la période est appliquée de façon continue sur une pluralité de cycles consécutifs du signal électrique SG1d. Cette variation peut par exemple être linéaire, ou non linéaire, au cours du temps.

La figure 13 représente dans cet exemple le signal électrique SG1d modulé, où le temps est représenté selon l'axe des abscisses (en nombre de cycles d'horloge) et la tension du signal est représentée selon l'axe des ordonnées. Comme illustré dans cet exemple, la période du signal électrique SG1d est augmentée progressivement au cours du temps sur une pluralité de cycles.

L'application d'une telle variation 60 de période permet avantageusement de minimiser les harmoniques contenues dans le signal électrique SG1d (et donc dans les ondes ultrasonores W en sortie) et ainsi de limiter le rayonnement EM causé par ces harmoniques.

Plus spécifiquement, comme illustré en figure 14, le spectre fréquentiel 64 du signal électrique SG1d comprend par exemple un pic principal 65 à une fréquence fondamentale et des pics secondaires 66a, 66b, 66c et 66d (notés collectivement 66) à des fréquences respectives d'harmoniques. La modulation ML1 par variation de la période dans cet exemple permet de réduire sensiblement les harmoniques 66, notamment l'harmonique 66b (multiple de 7 de la fréquence fondamentale), et ce tout en maintenant à un niveau élevé le pic 65 à la fréquence fondamentale.

En particulier, la diminution des harmoniques 66 résulte d'un élargissement spectral causé par la variation 40 de la période qui perturbe la périodicité du signal SG1d. Comme illustré, on obtient en effet une multiplication des pics en fréquence contenus dans le signal SG1d par rapport au cas où aucune modulation ML1 n'est appliquée, ce qui se traduit par une redistribution de l'énergie électrique dans le spectre fréquentiel du signal SG1b.

Ainsi, la modulation ML1 appliquée au cours de l'étape E2 (figure 6) peut donc être réalisée de diverses manières, les modes de réalisation ci-dessus n'étant décrits qu'à titre d'exemple.

Selon un exemple, la modulation ML1 (étape E2, figure 6) de la phase PH1 est configurée pour que le signal électrique SG1 délivre une énergie électrique E2 au moins égale à une énergie théorique E1 qui serait délivrée par le signal électrique SG1 si sa phase PH1 n'était pas perturbée par ladite modulation de phase ML1 (E2 ≥ E1). En respectant ce principe de conservation de l'énergie électrique, on peut avantageusement obtenir des performances satisfaisantes, en termes notamment d'énergie (ou puissance) acoustique délivrée en sortie du dispositif ultrasonore 20. On peut ainsi limiter avantageusement le niveau des émissions électromagnétiques avec un minimum d'impact (voire aucun impact) sur les performances de fonctionnement souhaitées du dispositif ultrasonore 20.

Selon un exemple, la modulation ML1 (étape E2, figure 6) de la phase est réalisée tout en conservant une même fréquence f sur chaque cycle du signal électrique SG1.

Comme le comprend l'homme du métier, tous les modes de réalisation et variantes décrits ci-avant dont certains ont été simplifiés à dessein pour faciliter les explications, ne constituent que des exemples non limitatifs de mise en oeuvre de la présente divulgation. En particulier, l'homme du métier pourra envisager une quelconque adaptation ou combinaison des modes de réalisation et variantes décrits ci-avant, afin de répondre à un besoin particulier.

La présente divulgation ne se limite donc pas aux exemples de réalisation décrits ci-avant mais s'étend notamment à un procédé de traitement qui inclurait des étapes secondaires sans pour cela sortir de la portée de la présente divulgation. Il en serait de même d'un système de traitement pour la mise en oeuvre d'un tel procédé.

## Revendications

1. Procédé de traitement d'un signal électrique (SG1) pour un dispositif émetteur ultrasonore (20), ledit procédé comprenant :

   - génération (E2) d'un signal électrique (SG1) définissant des ondes ultrasonores (W), la périodicité du signal électrique étant modifiée par une modulation (ML1) de la phase (PH1) du signal ; et
   - fourniture (E4) du signal électrique (SG1) au dispositif émetteur ultrasonore (20) pour causer l'émission des ondes ultrasonores (W).

2. Procédé selon la revendication 1, dans lequel la modulation (ML1) de la phase (PH1) du signal électrique comprend :

   - introduction, dans le signal électrique (SG1a), d'au moins un délai (30) correspondant à une période pendant laquelle la tension du signal électrique est maintenue à une valeur constante.

3. Procédé selon la revendication 2, dans lequel ledit au moins un délai (30) est strictement inférieur à une demi-période du signal électrique.

4. Procédé selon la revendication 2 ou 3, dans lequel au moins 4 délais (30) sont introduits dans le signal électrique.

5. Procédé selon l'une quelconque des revendications 2 à 4, dans lequel le signal électrique comprend une pluralité de séquences successives comprenant chacune au moins un cycle ondulatoire du signal électrique, un délai séparant chaque paire de séquences consécutives, le signal électrique généré au cours desdites séquences étant en phase.

6. Procédé selon l'une quelconque des revendications précédentes, dans lequel la modulation (ML1) de la phase (PH1) du signal électrique comprend :

   - un décalage angulaire (40 ; 50) de la phase du signal électrique (SG1b, SG1c) dans au moins un cycle du signal électrique.

7. Procédé selon la revendication 6, dans lequel le décalage angulaire (40) est configuré pour causer une inversion de la phase (PH1) du signal électrique (SG1b).

8. Procédé selon la revendication 6 ou 7, dans lequel un décalage angulaire (50) est introduit à chaque demi-période du signal électrique (SG1c).

9. Procédé selon la revendication 8, dans lequel le signal électrique modifié par ladite modulation comprend au moins l'une quelconque parmi :

   - au moins une première phase au cours de laquelle le décalage angulaire introduit dans le signal électrique croît progressivement ; et
   - au moins une deuxième phase au cours de laquelle le décalage angulaire introduit dans le signal électrique décroît progressivement.

10. Procédé selon l'une quelconque des revendications précédentes, dans lequel la modulation (ML1) de la phase (PH1) du signal électrique comprend une variation (60) de la période des cycles du signal électrique (SG1d) au cours du temps.

11. Procédé selon l'une quelconque des revendications précédentes, dans lequel la modulation (ML1) de la phase est configurée pour que le signal électrique (SG1) délivre une énergie électrique au moins égale à une énergie théorique qui serait délivrée par le signal électrique si sa phase n'était pas perturbée par ladite modulation de phase.

12. Procédé selon l'une quelconque des revendications précédentes, dans lequel la modulation (ML1) de la phase (PH1) est réalisée tout en conservant une même fréquence sur chaque cycle du signal électrique (SG1).

13. Procédé selon l'une quelconque des revendications précédentes, dans lequel les ondes ultrasonores (W) sont des ondes de compression engendrant des ondes de cisaillement dans un milieu (M).

**14.** Procédé selon l'une quelconque des revendications précédentes, dans lequel ledit procédé est appliqué à l'imagerie médicale par ultrasons.

**15.** Programme d'ordinateur (PG1) comportant des instructions pour l'exécution des étapes d'un procédé selon l'une quelconque des revendications précédentes lorsque ledit programme est exécuté par un ordinateur (10).

**16.** Dispositif (10) de traitement d'un signal électrique (SG1) pour un dispositif émetteur ultrasonore (20), ledit dispositif de traitement comprenant :

- un module de génération (MD2) configuré pour générer un signal électrique (SG1) définissant des ondes ultrasonores (W), la périodicité du signal électrique étant modifiée par une modulation (ML1) de la phase (PH1) du signal ; et
- un module de fourniture (MD4) configuré pour fournir le signal électrique au dispositif émetteur ultrasonore (20) pour causer l'émission des ondes ultrasonores (W).

## Fig.1

## Fig.2

# Fig.3

# Fig.4

# Fig.5

MD2: ML1

MD4 → SG1
(PH1)

10

# Fig.6

E2

E4

ML1

SG1
(PH1)

W

# Fig.7

# Fig.8

**Fig.9**

**Fig.10**

# Fig.11

ML1

SG1c
(PH1)

50

# Fig.12

54

all

56

55

56a    56b    56c

fondamental

55

harmonic 7

56c

## Fig.13

SG1d
(PH1)

60

ML1

## Fig.14

64

all 66

65

66b 66c

66a

66d

dB

0   10  20  30  40  50  60
MHz

fondamental

65

dB

3.5  4  4.5  5  5.5
MHz

harmonic 7

66c

dB

30.5  31  31.5  32  32.5
MHz

Europäisches Patentamt
European Patent Office
Office européen des brevets

# RAPPORT DE RECHERCHE EUROPEENNE

Numéro de la demande

EP 24 18 6943

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | GB 2 593 477 A (UNIV WARWICK [GB]) 29 septembre 2021 (2021-09-29) <br><br> * abrégé *; figures 1-13 * <br> * page 1, ligne 4 - page 4, ligne 32 * <br> * page 6, ligne 6 - page 13, ligne 31 * <br> ----- | 1,2,5-7, 11,12, 15,16 | INV. G01S7/52 B06B1/02 |
| X | US 2015/348531 A1 (FREEAR STEVEN [GB] ET AL) 3 décembre 2015 (2015-12-03) | 1-4,6-8, 10,11, 13-16 | |
| Y | * abrégé *; figures 1-3, 17-26, 43 * <br> * alinéa [0001] - alinéa [0042] * <br> * alinéa [0084] - alinéa [0156] * <br> * alinéa [0204] - alinéa [0209] * <br> ----- | 9 | |
| X | ZHOU YUFENG: "The Effects of Phase-Modulated Excitation on the Focused Acoustic Field", IEEE TRANSACTIONS ON ULTRASONICS, FERROELECTRICS, AND FREQUENCY CONTROL, IEEE, USA, vol. 67, no. 4, 26 novembre 2019 (2019-11-26), pages 727-734, XP011780140, ISSN: 0885-3010, DOI: 10.1109/TUFFC.2019.2955453 [extrait le 2020-03-25] * le document en entier * <br> ----- | 1,15,16 | |
| | | | DOMAINES TECHNIQUES RECHERCHES (IPC) |
| | | | G01S B06B |
| Y | US 2004/254459 A1 (KRISTOFFERSEN KJELL [NO] ET AL) 16 décembre 2004 (2004-12-16) * abrégé *; figures 1-10 * * alinéa [0043] - alinéa [0053] * <br> ----- | 9 | |

Le présent rapport a été établi pour toutes les revendications

| Lieu de la recherche | Date d'achèvement de la recherche | Examinateur |
|---|---|---|
| Munich | 22 novembre 2024 | Zaneboni, Thomas |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 24 18 6943

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-11-2024

| Document brevet cité au rapport de recherche | Date de publication | Membre(s) de la famille de brevet(s) | Date de publication |
|---|---|---|---|
| GB 2593477 A | 29-09-2021 | AUCUN | |
| US 2015348531 A1 | 03-12-2015 | EP 2934770 A2 | 28-10-2015 |
| | | US 2015348531 A1 | 03-12-2015 |
| | | WO 2014096789 A2 | 26-06-2014 |
| US 2004254459 A1 | 16-12-2004 | DE 102004027025 A1 | 30-12-2004 |
| | | JP 4942290 B2 | 30-05-2012 |
| | | JP 2005000663 A | 06-01-2005 |
| | | US 2004254459 A1 | 16-12-2004 |

EPO FORM P0460

Pour tout renseignement concernant cette annexe : voir Journal Officiel de l'Office européen des brevets, No.12/82